# EUROPEAN PATENT APPLICATION

(11) **EP 4 821 426 A1**
(43) Date of publication of application: **14.10.2026**
(21) Application number: 26150029.2
(22) Date of filing: 02.01.2026
(51) Int. Cl.: A61B 5/00

(54) **MATERNAL FETAL MONITORING SYSTEM AND METHOD**

(30) Priority: 16.01.2025 US 202519025211
(71) Applicant: GE Precision Healthcare LLC, Waukesha, WI 53188 (US)
(72) Inventor: VENUGOPALAN, Vijith, Waukesha, 53188 (US); KESSLER, Dianne, Waukesha, 53188 (US); PILLI, Naga Durga Purnima, Waukesha, 53188 (US)
(74) Representative: Kilburn & Strode LLP

(57) **Abstract**

A maternal-fetal monitoring system comprises at least one physiological sensor configured to obtain at least a first maternal parameter of a maternal patient and at least a first fetal parameter of a first fetal patient. A controller is configured to compare the first fetal parameter to a plurality of fetal alarm thresholds and generate a first fetal alarm for the first fetal patient in response to the first fetal parameter passing a first one of the fetal alarm thresholds, wherein the first fetal alarm includes a first fetal alarm time and a first fetal alarm level. A new fetal alarm is generated each time the first fetal parameter passes one of the plurality of fetal alarm thresholds, wherein each new fetal alarm includes a new fetal alarm time and a new fetal alarm level. A list of fetal alarms is generated for the first fetal patient based on the first fetal alarm and each new fetal alarm.

## Description

### BACKGROUND

The present disclosure generally relates to patient monitoring systems, and specifically to patient monitoring systems for monitoring maternal and fetal patients, such as during labor and delivery.

Medical devices are known that can be used to detect a fetal electrocardiogram (fECG) without making physical contact with the fetus. One common solution for monitoring maternal and fetal patients includes the use of ultrasound to detect fetal heart rate (fHR) and maternal heart rate (mHR) and a tocodynamometer to detect uterine activity. Alternatively or additionally, systems may use electrodes that are placed on the mother's skin to detect electrophysiological signals, including fHR. The maternal heart signal will also tend to be detected by the electrodes. Given the amplitude of the maternal cardiac signal compared to the amplitude of the fetal cardiac signal, it can be challenging to separate the fetal heart signal from the maternal heart signal. The electrical signals detected by the electrodes can be processed to determine: the fetal heart rate (from the fECG), the maternal heart rate (from the mECG). Maternal contractions, often referred to as uterine activity (UA) can be determined by electrohysterography (changes in electrical potential due to uterine contractions), which can be detected by the electrodes. Alternatively or additionally, UA can be detected by a tocodynamometer, a strain gauge, or by other means.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the disclosure, a maternal-fetal monitoring system configured to monitor physiological parameters of a maternal patient and at least a first fetal patient, the system comprising at least one physiological sensor configured to obtain at least a first maternal parameter of the maternal patient and at least a first fetal parameter of the first fetal patient. A controller is configured to compare the first fetal parameter to a plurality of fetal alarm thresholds and generate a first fetal alarm for the first fetal patient in response to the first fetal parameter passing a first one of the fetal alarm thresholds, wherein the first fetal alarm includes a first fetal alarm time and a first fetal alarm level. A new fetal alarm is generated each time the first fetal parameter passes one of the plurality of fetal alarm thresholds, wherein each new fetal alarm includes a new fetal alarm time and a new fetal alarm level. A list of fetal alarms is generated for the first fetal patient based on the first fetal alarm and each new fetal alarm.

In another aspect of the disclosure, a method of monitoring maternal and fetal patients comprises obtaining at least a first maternal parameter of the maternal patient and at least a first fetal parameter of the first fetal patient, comparing the first fetal parameter to a plurality of fetal alarm thresholds, and generating a first fetal alarm for the first fetal patient in response to the first fetal parameter passing a first one of the fetal alarm thresholds, wherein the first fetal alarm includes a first fetal alarm time and a first fetal alarm level. A new fetal alarm is then generated for each time the first fetal parameter passes one of the plurality of fetal alarm thresholds, wherein each new fetal alarm includes a new fetal alarm time and a new fetal alarm level. A list of fetal alarms is generated for the first fetal patient based on the first fetal alarm and each new fetal alarm.

Various other features, objects, and advantages of the invention will be made apparent from the following description taken together with the drawings.

### BRIEF DESCRIPTION OF THE DRAWINGS

The present disclosure is described with reference to the following Figures.
FIG. 1 is a system diagram of an exemplary embodiment of a system for monitoring a maternal patient and a fetal patient according to the present disclosure.
FIG. 2 is a layout diagram of an exemplary embodiment of a patch for monitoring fetal and maternal physiological parameters.
FIG. 3 is a perspective view of another exemplary embodiment of a patch and readout device.
FIG. 4 is a block diagram of an exemplary embodiment of a readout device according to one embodiment of the present disclosure.
FIG. 5 is a flow diagram illustrating one embodiment of a method of monitoring maternal and fetal patients according to one embodiment of the present disclosure.
FIG. 6 illustrates an exemplary detection of fetal alarms and corresponding generated list of fetal alarms according to one embodiment of the present disclosure.
FIG. 7 illustrates an exemplary detection of fetal alarms and corresponding generated list of fetal alarms according to another embodiment of the present disclosure.
FIG. 8 illustrates another user interface view providing the list of fetal alarms shown in FIG. 7, according to another embodiment of the present disclosure.
FIG. 9 illustrates another exemplary detection of fetal alarms for physiological parameter with unlatched alarming according to another embodiment of the present disclosure.
FIG. 10 illustrates another user interface view providing a list of fetal alarms according to another embodiment of the present disclosure.
FIG. 11 is a flow diagram illustrating one embodiment of a method of monitoring two fetal parameters of a fetal patient according to another embodiment of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

Current maternal-fetal monitoring systems provide threshold alarms based on the physiological value of parameters like fetal heart rate, maternal heart rate, SPO2, blood pressure etc. wherein a maximum of one alarm is triggered when a measured value for a physiological parameter is outside a specified range. This results in a single alarm with a single priority level based on the last-breached threshold. Often, the clinician will delay in responding to the alarm, by which time the alarm status has changed and the old alarm status is lost. Thus, the clinician may miss the fact that other alarms were previously generated for a given parameter because those alarming values have resolved themselves or have escalated to a higher alarm level.

The present disclosure is disclosure aims at creating a more thorough alarm reporting where multiple alarms can be generated and presented for a single physiological parameter of a single patient. A list of alarms are generated for each patient containing multiple alarms for that patient, including multiple alarms for a single physiological parameter. Thus, where a fetal patient and a maternal patient are both being monitored, a separate list of alarms may be generated for the fetal patient and another for the maternal patient. In other embodiments, only a list of fetal alarms may be generated and the alarms for the maternal patient may be tracked and displayed by standard means where only one alarm is generated per physiological parameter and only the current alarm information is displayed.

Where multiple fetal patients are being monitored (such as with twins or triplets), a separate list of fetal alarms is generated for each fetal patient. Where multiple latched parameters are monitored for a given patient, such as a given fetal patient, then the list of alarms may include multiple alarms for each latched parameter. In some embodiments, a maximum number of alarms may be set for each parameter, where the list contains up to the maximum number of alarms for that physiological parameter. The maximum number of alarms may be different for each parameter with latched alarms, or the maximum number of alarms may be the same for each parameter with latched alarms. Alternatively or additionally, the maximum number of alarms may be a user-configurable value.

In some embodiments, the system is configured to order the list of alarms for one or more of the patients, such as based on alarm priority and/or alarm time. In one such embodiment, the list of alarms is ordered first based on priority and then based on alarm time, such as providing the alarms in descending time order with the most recent alarm in each priority category being listed first and the oldest alarm in that category listed last.

Various monitoring solutions are available for monitoring maternal and fetal patients to obtain fHR, mHR, uterine activity, as well as other physiological parameters of one or both patients, including SpO2, blood pressure, and/or temperature. One common solution for monitoring maternal and fetal patients includes the use of ultrasound to detect fetal heart rate (fHR) and maternal heart rate (mHR) and a tocodynamometer to detect uterine activity. Electrode based solutions are also available. For example, biopotential maternal and fetal monitors exemplarily use five silver/silver chloride wet electrodes to collect biopotentials from which measurements of fHR, mHR, as well as uterine activity are derived by computer processing of the collected bio potentials which include electrocardiographic data of both the maternal and fetal patients as well as uterine electromyographic data from which contractions are identified. Uterrine activity (UA) can be determined by the electrical signals obtained via the electrodes. Alternatively or additionally, UA can be detected by a tocodynamometer, a strain gauge, or by other means.

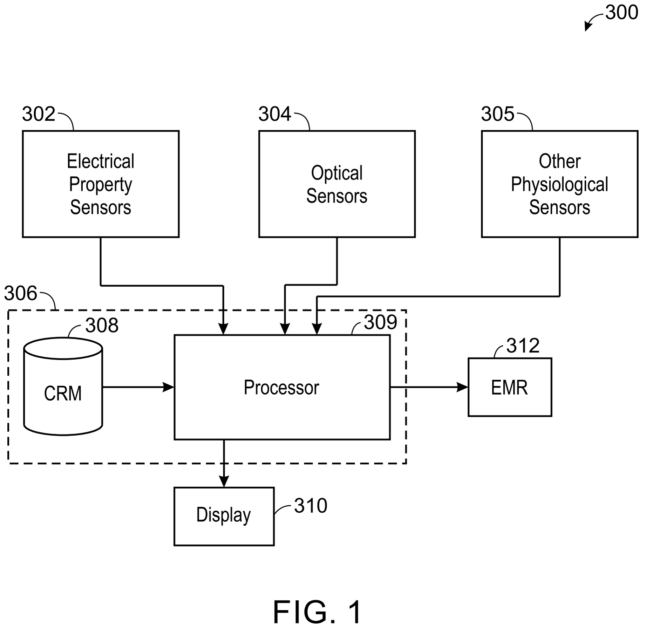

The following figures illustrate embodiments of the maternal-fetal monitoring system; however, the disclosed invention may be implemented with any of various sensing devices and methods for obtaining maternal and fetal physiological parameter measurements. For example, whereas FIGS. 1-4 illustrate systems implementing electrodes for obtaining fetal and maternal cardiac signals, other embodiments may implement one or more ultrasound transducers, tocodynamometers, or other sensing devices without the use of electrodes (or in combination with electrodes). Similarly, where FIGS. 1-4 illustrate systems implementing electrodes for obtaining UA, other embodiments may be configured to utilize a tocodynamometer to obtain UA. Still other embodiments for obtaining maternal and fetal physiological parameters are known and withing the scope of the present disclosure.

FIG. 1 illustrates an exemplary maternal-fetal monitoring system 300 for monitoring both maternal and fetal patients. The system 300 is provided in schematic form and it will be recognized by a person of ordinary skill in the art that such system 300 may be implemented in a variety of manners, including the distribution and interconnection of components represented in FIG. 1. Various components may be provided in one or more physical devices while being communicatively connected generally in the manner as shown in FIG. 1. As noted above, the system 300 includes electrical property sensors 302, for example, biopotential electrodes configured to collect biopotentials from the abdominal skin of the maternal patient, which include biopotential signals from both the maternal and fetal patients. The system 300 also includes one or more optical sensors 304 and/or other physiological sensors 305. The optical sensors 304 and other auxiliary sensors 305 are used to obtain signals indicative of mechanical motion by the maternal and/or fetal patients, for example but not limited to, heart contractions, blood flow, or uterine contractions. The optical sensor may be configured to measure optical parameters used for the calculation of SpO₂ for the maternal and/or fetal patient(s). Optical signals can also be affected by tissue absorbance of photon energy due to metabolite content, for example Lactate, or blood gas content, for example Oxygen.

Other auxiliary sensors 305 may include ultrasound sensor(s), tocodynamometer(s), microphone(s), accelerometer(s), piezoelectric sensor(s), strain gauge(s), and other sensors that will be recognized by a person of ordinary skill in the art as configured to monitor maternal and/or fetal physiological parameters, such as configured for use on a maternal patient during labor and delivery. Auxiliary sensors 305 may include sensors configured to monitor only the fetus, such as intrauterine fetal monitoring devices. Alternatively or additionally, auxiliary sensors 305 may include sensors configured to monitor maternal physiological parameters, such as a non-invasive blood pressure (NIBP) sensor configured to measure maternal blood pressure, a pulse oximetry sensor configured to monitor maternal SpO₂, one or more chest electrodes configured to monitor maternal ECG, or the like.

The signals obtained by the electrical property sensors 302 and the optical sensors 304 and any other auxiliary sensors 305 are provided to a controller 306. The controller 306 may be any of a variety of controllers, microcontrollers, processors, or integrated circuits as known in the art of physiological sensing for collection and/or analysis of physiological data. The controller 306 is configured to collect data from the various sensors 302, 304, 305. In embodiments where the data is collected in analog format, an internal or external analog-to-digital converter, such as an analog front end, is configured to digitize the physiological signals. Here, the controller 306 includes at least one processor 309 is communicatively connected to a computer readable medium (CRM) 308 which is a non-transitory computer readable medium or media upon which computer readable code embodying software programs containing algorithm and/or software modules containing algorithms which upon execution by the processor(s) 309 cause it to carry out the calculations and functions as described in further detail herein. The CRM 308 may be internal or external to the processor 309. In some embodiments, the controller 306 may include several processors, CRMs, and/or other computing devices communicatively connected, which may be housed together or distributed across several networked devices, such as in a distributed network managed by a healthcare provider. In exemplary embodiments, the software and software modules as executed by the controller 306 are such as to isolate and analyze particular signals within the signals obtained from the electrical property sensors 302 as well as those obtained from the optical sensors 304 and any other auxiliary sensors to execute various alarming and other patient monitoring functions as described herein based upon the isolated and analyzed signals from the electrical property sensors 302, optical sensors 304, and any other auxiliary sensors 305. The controller 306 provides alarm notifications along with the physiological parameter values and information to a display 310 which may operate to present one or more of the calculated values in a visually perceptible manner or stored in a patient electronic medical record (EMR) 312. The controller 306 may provide the calculated physiological parameter values across a wired or wireless connection and therefore, the display 310 or the EMR 312 may be physically connected to the controller 306 or may be located remote from the controller 306.

As noted above, it will be recognized that in embodiments, the electrical property sensors 302 and the optical sensors 304 and any other auxiliary sensors 305 may be integrated into a single device configured to be attached to the maternal patient, such as integrated into the same patch of sensors as will be described in further detail herein, or may be provided as separate components. Additionally, the controller 306 may be integrated with such a patch containing the electrical property sensors 302 and the optical sensors 304 while in other embodiments, the system 300 may include additional instrumentation such as one or more wireless transmitters configured to transmit the signals acquired from the electrical property sensors 302 and the optical sensors 304 and any other auxiliary sensors 305 to be controller 306 which may be located remotely from the sensor. Alternatively or additionally, the controller 306 may include one or more wireless transmitters configured to transmit the patient monitoring data to a healthcare facility network, such as for transmission to a remote monitoring station for viewing by a clinician and/or storage in the EMR 312.

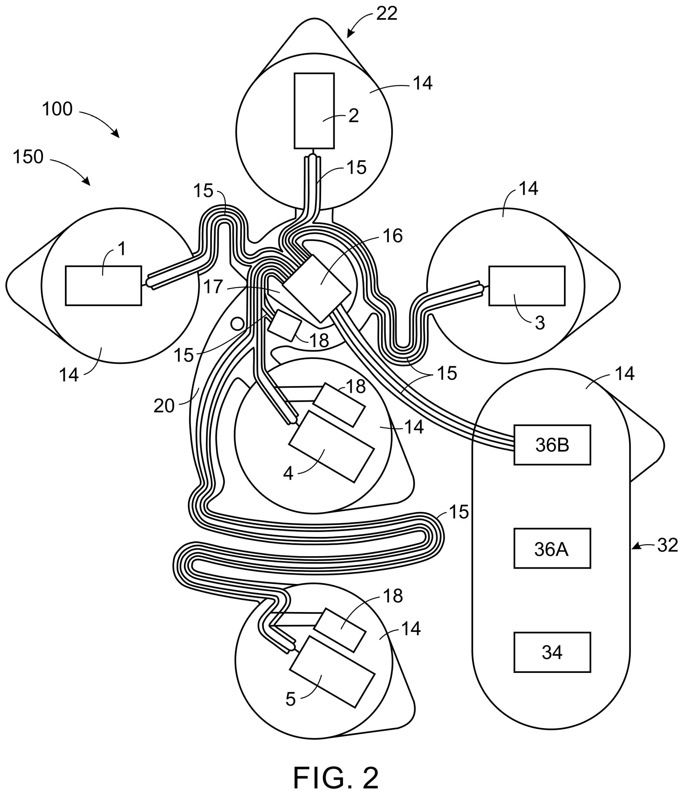

Referring to FIG. 2, a patch 150 exemplary of embodiments is shown. The patch 150 includes a flexible substrate 100, viewed from the side that is to be facing the abdomen, in use. The flexible substrate 100 forms electrodes 1-5. Each electrode 1-5 is connected via a conducting track 15 to a connection hub 16, for electrically connecting electrodes 1-5 to a readout device (not shown). In embodiments as represented in FIG. 1, the substrate 100 may further be shaped to provide for a plurality of sensors, including, and in addition to electrodes 1-5. The plurality of sensors, as described in further detail herein may include at least one auxiliary sensor 18, although in embodiments more auxiliary sensors 18 may be found in the plurality of sensors. In embodiments, the conducting track 15 may be provided to electrically connect the one or more auxiliary sensors 18 to the connection hub 16. The one or more auxiliary sensors 18 may include one or more types of sensors configured to produce a signal indicative of a physiological parameter and/or the mechanical motion of the fetal patient and/or the maternal patient. As noted above, in exemplary and non-limiting embodiments, the auxiliary sensor 18, may be any of a strain gauge, a piezoelectric sensor, an accelerometer, a microphone, temperature sensor, SpO2 sensor, an ultrasound Doppler, a capacitive micromachined ultrasonic transducer, or other sensors as will be recognized by a person of ordinary skill in the art in view of the present disclosure. Non-limiting embodiments of physiological parameters may include heart rate, SpO₂, ECG, blood pressure, breath rate, and/or UA. Non-limiting embodiments of mechanical motion of the fetal patient may include fetal motion and/or kick counting, or fetal heart rate as monitored by fetal heart sounds, fetal heart movement, or fetal blood movement. Non-limiting embodiments of mechanical motion of the maternal patient may include maternal respiration movement or sounds, maternal heart sounds, maternal blood movement or sound, and maternal uterine contractions.

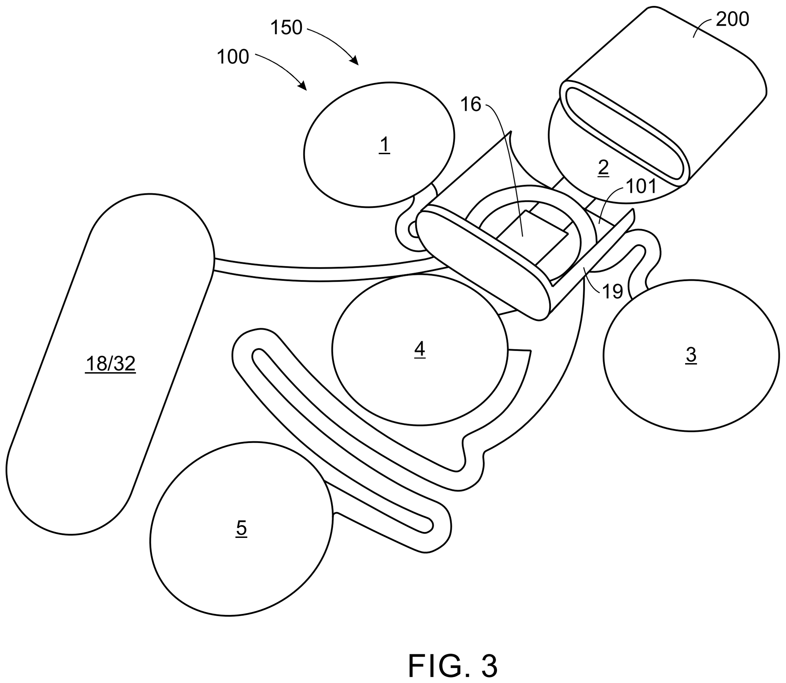

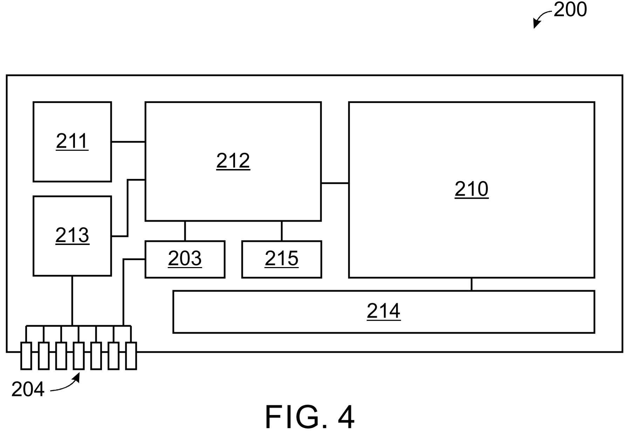

FIG. 2 provides three exemplary locations for the optical sensor 18 or other auxiliary sensors, these include placing the optical sensor 18 in one or more of the patch regions 21-25 which contain the electrodes 1-5, in a central patch region 20 of the substrate, or in a dedicated sensor patch region 26 of the substrate 100. However, it will be recognized that embodiments may include auxiliary sensors 18 including the optical sensor at one of these locations, at all of these locations, or at other locations. Such other locations may include, but are not limited to within the connection hub 16 or within the readout device 200 as will be described in further detail herein, for example with respect to FIGS. 3 and 4.

The substrate 100 comprises a reference feature 17, for lining up with an umbilicus or other suitably recognizable feature of the subject. In this case, the reference feature 17 is defined by an aperture in the flexible substrate 100. In other embodiments the reference feature 17 may be a vertex, pointer or transparent region forming in the flexible substrate 100. The reference feature 17 may be associated with an adjacent adhesive region, by which the reference feature 17 can be secured to the subject, for example adjacent to the umbilicus. As depicted in FIG. 2, the auxiliary sensor 18 may be located at a region about the reference feature 17. In embodiments when the reference feature 17 is centrally located to the patch regions 21-26, the region about the reference feature 17 may provide also provide a central and desirable location from which to measure the mechanical movement of the maternal patient and/or the fetal patient. In still further embodiments wherein an optical sensor and other auxiliary sensors 18 are used, an auxiliary sensor 18 located in the region of the reference feature 17 can be used as a reference sensor for example for noise cancellation or as an active electrode for injecting a mechanical, e.g. acoustic signal into the maternal and fetal patients.

The structure lends itself to a straightforward method of application. For example, the reference feature 17 may be secured at a reference point on the patient using the associated adhesive region. The electrodes 1-5 and respectively on regions 21-25 and an optical sensor or other auxiliary sensor 18 on region 26 can subsequently be moved away from the abdomen to prepare the skin. For example each patch regions 21-26 can then be placed in turn around the abdomen with, if necessary, suitable abrasive skin preparation. It will be recognized that while electrodes 1-5 may require a suitable electrode-skin interface 14, an auxiliary sensor may require such an interface, for example with a photoemitter/photosensor, an SpO₂ sensor, an ultrasound Doppler, a capacitive micromachined ultrasonic transducer, or a piezoelectric sensor. Other examples of auxiliary sensors, for example accelerometers, microphones, or strain gauges do not require the same electrical and/or acoustical interface with the maternal skin. Once any skin preparation, if needed, and the subsequent placement of an electrode 1-5 or auxiliary sensor has been completed the impedance of the connection between the electrode 1-5 and the patient may be measured by an electronic readout device 200 (shown in FIGS. 3 and 4). If the impedance is above a desired value, further preparation of the skin may be carried out to reduce the impedance to below the desired value. The desired value may, for example, be 5 kOhms. When the impedance is below the desired value, the skin region for the next electrode may be prepared by abrading the skin and the electrode subsequently applied electrode, and the impedance tested. This method may be repeated until all of the electrodes are successfully applied.

The optical sensor 18 may be located in a variety of positions relative to the rest of the patch 150. In one implementation, the detection of PPG and/or SpO₂ in photosignals requires alignment of the optical sensors with the placenta which provides the most blood pooling from the fetus blood in the uterus. The placenta may be in a similar alignment with the positioning of the biopotential sensors of the patch 150 or can be variable in its position during a pregnancy. The placenta may be either in the upper, middle, or lower portions of the uterus and may extend vertically or horizontally. Therefore, in examples, while the auxiliary sensors 18 embodying optical sensors may be integrated into the regions 21-25 adjacent to respective electrodes 1-5, in still further examples, a separate region 26 is provided to include the optical sensor 18, which for example, includes at least one photoemitter 34 and at least one or at least two photodetectors 36. The separate region 26, while it may be electrically or otherwise communicatively connected to the connection hub 16. The separate region 26 may be positioned relative to the location and orientation of the placenta for each patient and the separate region 26 provides this flexibility to tune the positioning of the region 26 to each patient. Further, the separate region 26 may be physically separate from the rest of the patch 150 and communicatively connected to provide the detected photosignals as described in further detail herein.

In one example implementation, the optical sensor is a reflective SpO₂ sensor 32 affixed to the abdomen of a pregnant patient. The emitter 34 (or emitters) projects light energy into the abdomen of the pregnant patient. The emitter 34 projects light energy within specific wavelengths. In one example, the light energy is visible light. In another example, the light energy is red and infra-red wavelengths of light. In a still further example, red, infra-red, and green wavelengths of light are used. Other wavelengths or combinations of wavelengths, which may be visible or not visible to the human eye, will be recognized by a person of ordinary skill in the art based upon the present disclosure. It will be recognized that various combinations of wavelengths or wavelength bands may be used, such combinations could include using light at one, three, five, seven or other numbers of wavelengths or wavelength bands. Light reflected back to the same skin surface as the emitter returns at different distances from the emitter 34 relative to the depth of the anatomical structure from which the light energy reflects. Photodetectors 36A and 36B are spaced apart from the emitter 34 at respective distances. In one embodiment, at least one of the distances corresponds to light reflected from the uterine wall of the patient, while at least one other of the distances corresponds to light reflected from the fetus within the uterus. In this manner, the signal obtained at one of the photodetectors, e.g., photodetector 36B, contains both maternal and fetal signals while the shallower reflected signal returned at another photodetector, such as photodetector 36A, contains only signals from the maternal patient.

FIG. 3 shows a top view of another exemplary embodiment of a patch. In this embodiment, the mechanical module unit 19 is affixed to the substrate 100 adjacent to the electrical module unit 16, as previously shown in FIG. 2. The patch 150 is shown with a separate electronic readout device 200 for detecting signals from the electrodes 1-5 and at least one auxiliary sensor 18 and/or optical sensor 32 mounted on the patch 150. In embodiments shown also in FIG. 4, the readout device 200 may include a processor or other processing electronics to analyze the detected signals and to produce physiological data of the fetal and maternal patients. In still further embodiments, the readout device 200 may rather serve a communicative function, providing wireless communication of the detected signals from the sensors of the patch 150 to a computer processor located remote from the patch 150. The remotely located computer processor may perform the same functions as described herein to analyze the detected signals to produce physiological data of the fetal patent and the maternal patient. In embodiments, the remotely located computer processor may be located in a same room as the maternal patient, while in other embodiments the remotely located computer processor may be a cloud or networked computer processing system wherein the detected signals are transmitted to a location further away for processing. In such embodiments, any calculated physiological data may be returned back locally for presentation to caregivers and/or the maternal patient.

The mechanical module 19 includes a cradle for receiving the readout device housing 201 of the readout device 200. When the readout device housing 201 is fully engaged with the mechanical module 19, an electrical module 204 (shown in FIG. 4) of the readout device 200 is in electrical engagement with the electrical module 16 of the patch 150. The electrical module 204 of the readout device 200 may conveniently comprise a plurality of contacts mounted on resiliently deformable members (e.g. spring loaded contact pins). The housing 201 is removably received and held within the cradle, which allows movement of the housing 201 only in the direction of insertion/removal relative to the cradle. The cradle comprises a stop, and the readout device 200 is fully engaged with the mechanical module unit 19 when the housing 201 is in contact with the stop. The mechanical module 19 further comprises a latch or catch to retain the readout device housing 201 in contact with the stop. In this embodiment, the latch or catch comprises a magnetic catch. A permanent magnet is provided on either (or both) of the mechanical module 19 and housing 201, which attracts a corresponding magnet (or ferromagnetic element) on the other of the mechanical module 19 or housing 201. In alternative embodiments, a hook and loop arrangement (e.g. Velcro) may be used to secure the readout device 200 to the patch 150. In an embodiment, the magnetic catch can be used to avoid incorrect positioning of the mechanical module.

In embodiments, the readout device 200 may also include one or more auxiliary sensors as described either above or in further detail herein. Auxiliary sensors may be located in the readout device 200, particularly those auxiliary sensors that may suitably operate without an acoustic or electric connection to the maternal patient's skin. Such sensors may exemplarily include an accelerometer. In other embodiments, the readout device 200 may include one or more additional auxiliary sensors, such as a microphone, a Doppler ultrasound sensor, or a capacitive micromachined ultrasonic transducer either to collect signals from the maternal patient or to collect ambient sounds for noise cancellation from signals obtained from a microphone arranged in the patch 150 to collect sounds of the maternal and fetal patients.

In further embodiments, additional functionality may be added to the patch device as described herein with the incorporation of additional types of auxiliary sensors either into the patch substrate as described with respect to FIG. 2 or incorporated into the readout device 200 as described with respect to FIG. 3. These additional auxiliary sensors may include, but are not limited to, a temperature sensor, a maternal SpO₂ sensor, or a motion sensor. A temperature sensor may be a thermistor or a thermocouple and when placed in contact with the maternal patient's skin can be used to provide an ongoing presentation of maternal temperature, for example to determine if the maternal patient is becoming too hot or too cold. The maternal SpO₂ sensor uses a small light emitting diode (LED) to project red and/or infra-red light into the patient to measure the amount of light in this spectrum that is absorbed, producing an indication of the Oxygen saturation of the patient's blood. Alternatively, the maternal SpO₂ sensor may be a finger-mounted pulse oximeter. In some embodiments, additional maternal physiological sensors may be included, such as an NIBP monitor configured to measure the maternal blood pressure. In some embodiments, additional fetal physiological sensors may be included, such as intra uterine devices configured to measure one or more fetal parameters directly from the fetus.

In still other embodiments, the maternal fetal monitoring system may comprise sensors configured to monitor multiple fetal patients (e.g., in the case of twins, triplets, etc.), such as two, three, four, or more fetal patients. The additional sensors may be duplicates of any of the sensors described above, such as multiple ultrasound sensors each configured to measure a heart rate of a respective feta patient in the maternal womb. Alternatively or additionally, some subset of sensors may be configured to obtain signals from multiple fetal patients. For example, the electrical activity recorded from the electrodes 1-5 may be processed to obtain information about two or more fetal patients in the maternal womb.

The readout device 200 may comprise one or more motion sensors 215 (FIG. 4), which may comprise an inertial sensor such as an accelerometer and/or gyroscope. The motion sensor 215 may comprise a one, two or three-axis accelerometer, and/or a one, two, or three axis gyroscope. The motion sensor 215 may include a MEMS (micro-electromechanical systems) devices or an inertial measurement unit. Motions sensors such as accelerometers and gyroscopes may be used to track the movement of the readout device 200, thereby allowing both fetal ECG and electrohysterogram (EHG) algorithms to differentiate between maternal/fetal movements and genuine contractions and fetal ECG signals and allowing to identify maternal pushing during the second stage of labor. A gyroscope may provide additional rotational information that an accelerometer cannot provide, thereby allowing further separation of fetal movement from the acquired data. This fetal movement may be a useful indicator that provides further fetal well-being indication. Additionally, the use of the pair of motion sensors allows separation of the maternal breathing signal which is a further indication of maternal health.

Referring to FIG. 4, a block diagram of a readout device 200 according to an embodiment is shown. The readout device 200 of FIG. 4 exemplarily presents a more detailed embodiment of the components of the controller as shown and described above with respect to FIG. 1. The readout device 200 comprises an electrical module unit 204, analog circuit 213, digital processor 212, wireless transmitter 211, security device 203, battery 210, and an inductive coil 214.

The analog circuit 213 includes an analog-to-digital converter (e.g., an analog front end), and receives the electrical signals from the electrodes and any electrical measurement signals from the mechanical movement sensors, and outputs a digitized version thereof, for processing by the digital signal processor. In some embodiments, the analog circuit 213 may include an amplifier and/or filter.

The processor 212 receives a digitized signal from the analog circuit 213, and preferably processes it to determine an output, as described already. The processor 212 subsequently outputs a signal to the wireless transmitter 211 for onward transmission, for example to a receiving and display station 310.

To maximize the battery life of the removable electronic device it may be configured such that the power of the wireless transmitter is controlled based upon the signal strength index and/or bit error rate. This may greatly lengthen the monitoring period that can be carried under one single battery charge.

The electronic components of the readout device 200 are powered by an electrical power source, such as a battery 210. In other embodiments the electrical power source may comprise a capacitor. The inductive coil 214 is operative to charge the battery 210 or to power the readout device directly, optionally under the control of the processor 212.

The readout device 200 may be configured to calculate an output at least one of an fHR, an fECG, an mHR, an mECG, UA and/or contraction strength. The readout device may further be configured to calculate an output of at least one of fPPG, mPPG, fSpO₂, and mSpO₂. Preferably the readout device is configured to output any combination or subset of the above. The readout device is preferably configured to transmit the output, so that it can be monitored according to the methods described herein. In other embodiments, the readout device 200 comprises part of the controller and control system that performs some or all of the methods described herein. In still other embodiments, does not calculate any of the physiological data as noted above, but rather transmits raw or partially processed voltage and/or current data from the sensors, for processing by a further device into a suitable output.

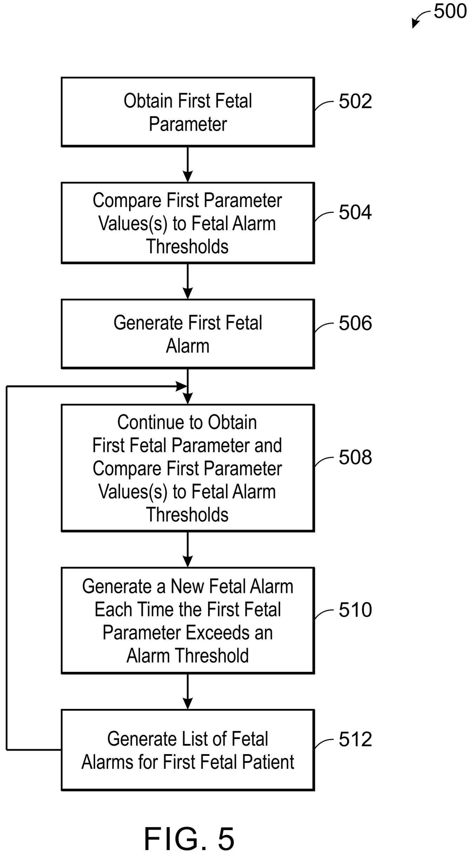

FIG. 5 shows one embodiment of a method 500 of processing the fetal physiological parameters for purposes of maternal and fetal monitoring. At least a first fetal parameter is obtained at step 502 for a first fetal patient. The first fetal parameter is monitored by the system using a latched alarming method as described herein, wherein an alarm must be acknowledged before it is resolved or removed, regardless of whether the first fetal parameter has changed and no longer meets the alarm condition. Further, each time the first fetal parameter passes a fetal alarm threshold or falls outside of a normal threshold range, a new alarm is generated. All of the previously generated and unacknowledged alarms are maintained, and thus the new alarm is added to the list of alarms.

In one embodiment, the first fetal parameter is fHR. In other embodiments, the first fetal parameter may be fetal SpO₂ or fetal blood pressure. In still other embodiments, multiple fetal parameters may be monitored as latched parameters according to the disclosed methods.

The first fetal parameter value is compared to one or more fetal alarm thresholds at step 504. The first fetal parameter may be compared to multiple fetal alarm thresholds, which may include, for example, a high threshold and a low threshold (where the values between the high and low thresholds are the normal range), a critical high threshold, and a critical low threshold. In other embodiments, three or more high or low thresholds may be assessed.

The first fetal alarm is generated at step 506 when the first fetal parameter passes (e.g., crosses above a high threshold or crosses below a low threshold) one of the fetal alarm thresholds. The first fetal alarm includes a fetal alarm time and a fetal alarm level. The fetal alarm time indicates the time that the first fetal alarm crosses the fetal alarm threshold, such as the time stamp of the first fetal parameter measurement (e.g., sample time) that passes one of the fetal alarm thresholds. The first fetal alarm level is based on the threshold passed. Where the system is configured as a multi-threshold, multi-alarm level system, the fetal alarm level indicates which of the alarm thresholds is passed. For example, a high fetal alarm level indicates that the high threshold is passed, whereas a critical high alarm level indicates that the critical high threshold is passed.

The first fetal parameter continues to be monitored, represented as step 508. Each time the first fetal parameter passes (e.g., crosses above a high threshold or crosses below a low threshold) one of the thresholds, a new fetal alarm is generated, as represented at step 510. Each new fetal alarm includes a new fetal alarm time and a new fetal alarm level indicating the alarm threshold crossed.

A list of fetal alarms is generated for the first patient at step 512. The list of fetal alarms contains the first fetal alarm and any new fetal alarm generated. In some embodiments, the list of fetal alarms may only include up to a maximum number of alarms for the first fetal parameter. In some embodiments, the list of fetal alarms may be a first-in-last-out type, where a newly generated fetal alarm replaces the oldest alarm for that fetal parameter on the list. Alternatively, the system may be configured to generate the list of fetal alarms to favor higher-priority alarms, and thus may be configured to remove a lower-priority (e.g., lower threshold) alarm from the list before removing an older critical alarm for that parameter.

In some embodiments, the system may be configured to execute the same process for another patient, including a maternal patient and/or one or more other fetal patients (e.g., in the case of twins or multiples). Thus, a list of maternal alarms may generated for the maternal patient, which may include multiple alarms for each latched maternal parameter for which the disclosed method is utilized. A maximum number of alarms for each latched maternal parameter may be included in the list, which may be the same maximum as used for the first fetal parameter or may be a different maximum value. Similarly, in the case of multiple fetuses, a separate list of fetal alarms may generated for each fetal patient, which may each include multiple alarms for each latched fetal parameter for which the disclosed method is utilized. A maximum number of alarms for each latched fetal parameter may be included in the list, which may be the same maximum as used for the first fetal parameter or may be a different maximum value. In some embodiments, the list of alarms (fetal and/or maternal) may include alarms for multiple latched parameters, and thus may include multiple alarms for each of two or more parameters.

In such an embodiment, the maximum number of alarms included may be the same for each latched parameter or a different maximum number value may be set for each parameter. Where the list is configured to only include a maximum number of fetal alarms for each latched fetal parameter or a maximum number of maternal alarms for each latched maternal parameter, the maximum number may be user configurable. In some embodiments, the system may be configured to facilitate the user setting one maximum number value to be used for all parameters. In another embodiment, the system may be configured to facilitate the user setting one maximum number value per patient-type, such as one for all fetal patients and one for the maternal patient. In still another embodiment, the system may be configured to facilitate the user setting a maximum number value per monitored fetal parameter and per monitored maternal parameter, where different maximum number values are set for each.

For instance, the system may be configured to enable a user to select a value from a predefined set of values to be used for the maximum number of alarms. The predefined set of values to be used for the maximum number (or each of several maximum numbers) may be between two predefined upper and lower limit values. For example, the lower limit value may be 2 and the upper limit value may be 15. In another embodiment, the lower limit value may be 3, may be 4, or may be 5. The upper limit value may be 12, or may be 10, or may be 8, or may be 5, or may be any value therebetween.

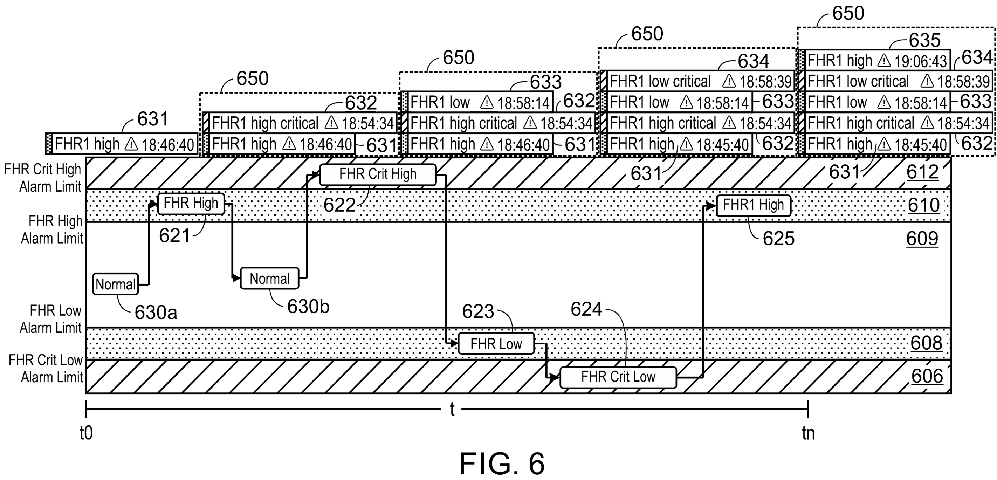

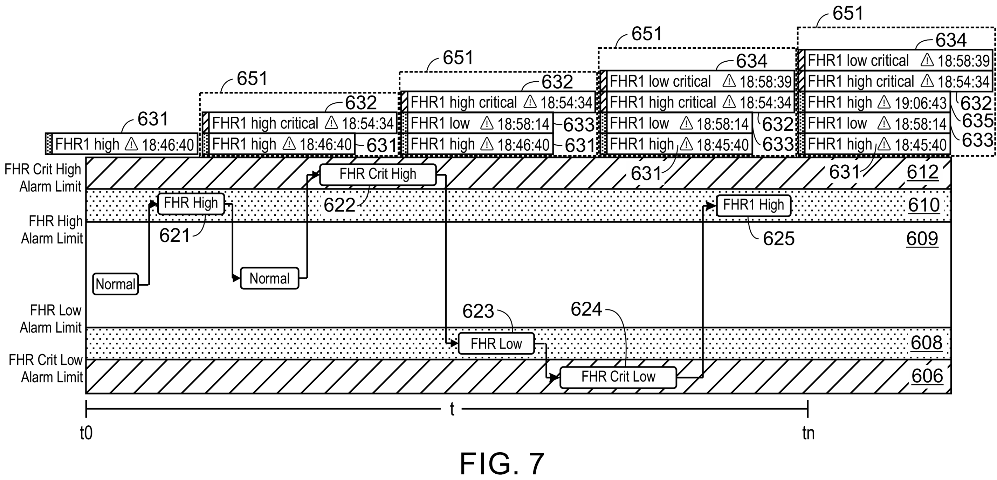

FIGS. 6 and 7 show embodiments of a fetal parameter collected over time and compared to a plurality of thresholds, where a list of alarms is generated documenting all of the alarms generated in a monitoring period (or at least up to a maximum number of alarms). Here, the first fetal parameter is fetal heart rate (fHR) monitored over time t between the start of monitoring (time t0) and the end of monitoring (time tn). In the exemplified arrangement, four fetal alarm thresholds are utilized that are alarm threshold values for fHR, including a high fHR alarm threshold 610, a critical high fHR alarm threshold 612, a low fHR alarm threshold 608, and a critical low alarm threshold 606. The values between the high fHR alarm threshold 610 and the low fHR alarm threshold 608 represent the normal range 609.

In the depicted scenario, five alarms are generated between time t0 and time tn. The fHR parameter value 630a starts out in the normal range 609. Thus no alarm is generated. The fHR parameter value 621 increases and passes the high fHR alarm threshold 610. A first fHR alarm 631 is generated, which includes an alarm time indicating the time that the fHR parameter value 621 increased passed the high alarm threshold 610 and a high alarm level indicating the threshold that was passed (here, the high threshold). The fHR parameter value returns to the normal range. However, the first alarm 631 remains and does not resolve without acknowledgment from a clinician, even though the fHR parameter value 630b is no longer exceeding the high fHR alarm threshold.

A second alarm 632 is generated when the fHR parameter value 622 passes the critical high alarm threshold 612. The second alarm 632 has an alarm time indicating when the fHR parameter value 622 passed the critical high alarm threshold 612. The second alarm 632 has a critical high alarm level indicating that the critical high alarm threshold 612 was surpassed. The first alarm does not resolve without acknowledgment from a clinician. Thus, both the first and second alarms are being generated simultaneously. The first list of fetal alarms 650 is generated containing the first and second alarms. Generation of alarms may include a visual alert on a display, a push notification alert to one or more caregivers, an auditory alert, or all of the above. In some embodiments, an auditory alert will be generated for only one of the alarms (e.g., the current or more recent alarm) and a visual alert is generated for all of the alarms.

A third alarm 633 is generated when the fHR parameter value 623 passes the low fHR alarm threshold 608. The first and second alarms do not resolve without acknowledgment from a clinician. The third alarm 633 has an alarm time indicating when the fHR parameter value 623 passed the low alarm threshold 608. The third alarm 633 has a low alarm level indicating the low fHR alarm threshold 608 was crossed. Thus, the first, second, and third are being generated simultaneously. The first list of fetal alarms 650 is updated to add the third alarm 633. Here, the first list of fetal alarms 650 is organized based on time only, where the latest alarm is at the top of the list and the earliest alarm is shown at the bottom.

A fourth alarm 634 is generated when the fHR parameter value 624 passes the critical low fHR alarm threshold 606. The first through third alarms do not resolve without acknowledgment from a clinician. The fourth alarm 634 has an alarm time indicating when the fHR parameter value 624 passed the critical low alarm threshold 606. The fourth alarm 634 has a critical low alarm level indicating the critical low fHR alarm threshold 606 was crossed. Thus, the first, second, third, and fourth are being generated simultaneously. The first list of fetal alarms 650 is updated to add the fourth alarm 634. The first list of fetal alarms 650 is organized based on time only, where the latest alarm is at the top of the list and the earliest alarm is shown at the bottom.

A fifth alarm 635 is generated when the fHR parameter value 625 passes the high fHR alarm threshold 610. The first four alarms do not resolve without acknowledgment from a clinician. The fifth alarm 635 has an alarm time indicating when the fHR parameter value 625 passed the high alarm threshold 610. The fifth alarm 635 has an alarm level indicating the high fHR alarm threshold was crossed. Thus, the first, second, third, fourth, and fifth alarms are being generated simultaneously. The first list of fetal alarms 650 is updated to add the fifth alarm 635. Thus, five alarms are listed. The first list of fetal alarms 650 is organized based on time only, where the latest alarm is at the top of the list and the earliest alarm is shown at the bottom.

The system may be configured to order the alarms in the list, and thus to generate an ordered list of alarms. The alarms may be ordered by time, alarm level, or both time and alarm level. FIGS. 6 and 7 show different embodiments of an ordered list of fetal parameters. As mentioned above, FIG. 6 illustrates an embodiment where the first list of fetal alarms 650 is ordered based on alarm time only, where the latest (most recent) alarm is at the top of the list and the earliest alarm is shown at the bottom. FIG. 7 illustrates an embodiment where the first list of fetal alarms 651 is ordered based on alarm level and alarm time. Here, the fetal alarms 631-635 are ordered based first on the respective fetal alarm level and then based on fetal alarm time. Thus, the most recent highest alarm level (e.g., critical level) is listed first, followed by any earlier alarms of that level (e.g., earliest critical alarms), followed by the most recent alarms of a lower criticality level (e.g., high and low alarms), followed by earlier alarms of that lower criticality level.

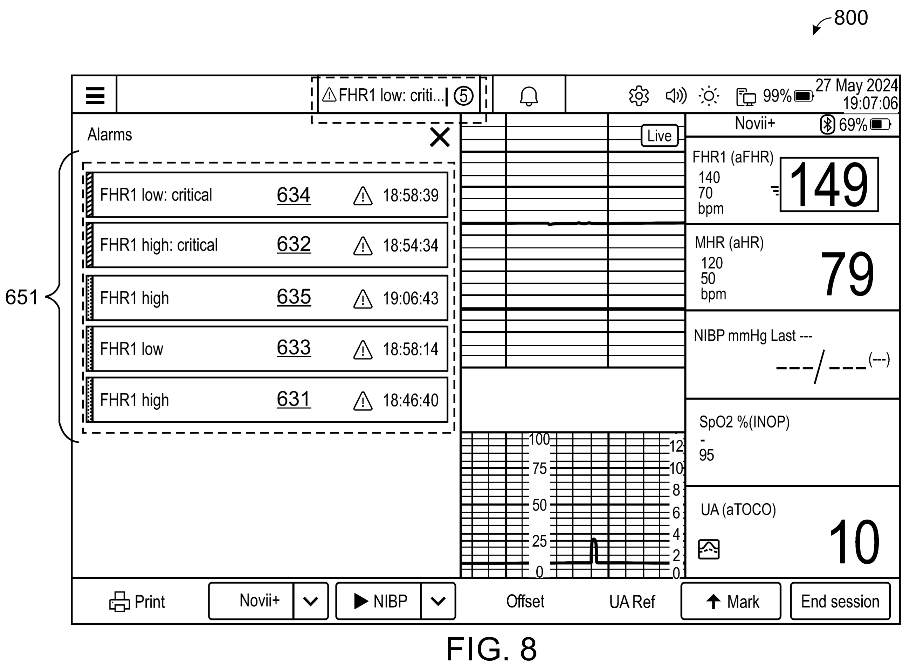

FIG. 8 illustrates another exemplary user interface 800 showing the ordered list of alarms for the fetal parameter for a given fetal patient. Here, the ordered list of alarms 651 is the same list illustrated in FIG. 7. The first list of fetal alarms 651 containing alarms for the first patient generated through time tn is ordered with the low critical alarm 634 listed first, since it is the most recent critical alarm. The high critical alarm 632 is listed next since it also has a critical value. The fHR high alarm 635 is next since it is the most recent alarm with the second-tier criticality level. Next the fHR low alarm 633 is listed since it is the next earliest alarm of that alarm tier. The fHR high alarm 631 is listed last since it is the oldest alarm and has the lowest criticality tier.

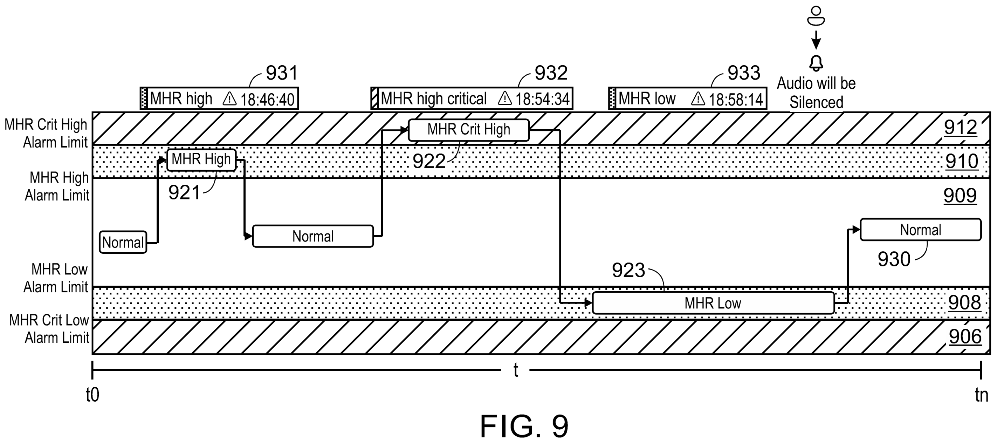

FIG. 9 shows an embodiment of alarm tracking of a physiological parameter with unlatched alarming. In contrast to latched alarming where an alarm condition does not resolve without user acknowledgment or other interaction by a user, an alarm generated according to an unlatched alarm regime will automatically resolve when the current parameter value is no longer in that value region (i.e., based on the alarm thresholds). Thus, for an unlatched parameter, a generated alarm is based on the current parameter value and only one alarm (maximum) will be generated for that physiological parameter at a given time.

In the example shown in FIG. 9, the physiological parameter with unlatched alarming is maternal heart rate (mHR). Since this physiological parameter is an unlatched parameter, a maximum of only one alarm for that parameter may be generated at a time. A first mHR alarm 931 is generated, which includes an alarm time indicating the time that the mHR parameter value 921 increased passed the high alarm threshold 910 and a high alarm level indicating the threshold passed (here, the high threshold). When the mHR parameter value returns to the normal range 909, the first alarm 631 resolves without acknowledgment from a clinician because the mHR parameter value no longer exceeds the high mHR alarm threshold.

A second maternal alarm 932 is generated when the mHR parameter value 922 passes the critical high alarm threshold 912. The second maternal alarm 932 has an alarm time indicating when the mHR parameter value 922 passed the critical high alarm threshold 912. The second maternal alarm 932 has a critical high alarm level indicating that the critical high alarm threshold 912 was surpassed. Like the first maternal alarm, the second maternal alarm resolves without acknowledgment from a clinician when the mHR parameter value 922 is no longer in the critical high range greater than the mHR critical high alarm threshold 912.

A third maternal alarm 933 is generated when the mHR parameter value 923 passes the critical high alarm threshold 913. The third maternal alarm 933 has an alarm time indicating when the mHR parameter value 923 passed the low alarm threshold 908. The third maternal alarm 933 has a low alarm level indicating that the low alarm threshold 908 was surpassed. The third maternal alarm 933 is maintained for the time that the mHR parameter value is in the low range between the low mHR alarm threshold 908 and the critical low mHR alarm threshold 906. Like the first and second maternal alarms, the third maternal alarm resolves without acknowledgment from a clinician when the mHR parameter value 930 is in the normal range, or is otherwise no longer in the low range. Thus, a maximum of one alarm is generated, and no alarm is generated when the mHR parameter value is in the normal range, even if previously the mHR parameter value was outside of the normal range so long as that condition resolved.

A list of alarms is generated containing the alarms for the maternal patient, including all of the alarms for all of the parameters associated with that patient. If there are latched alarms for maternal parameters monitored according to the disclosed method (e.g., as shown in FIGS. 6 and 7), then the list of alarms may contain multiple alarms for that latched parameter. Alarms for parameters with unlatched alarms ("unlatched parameters"), like the mHR shown in FIG. 9, are added to the list of alarms for that patient when they are generated and are removed from the list of alarms when the unlatched alarm automatically resolves. Thus, only one alarm for an unlatched parameter is ever on the list of alarms at a time.

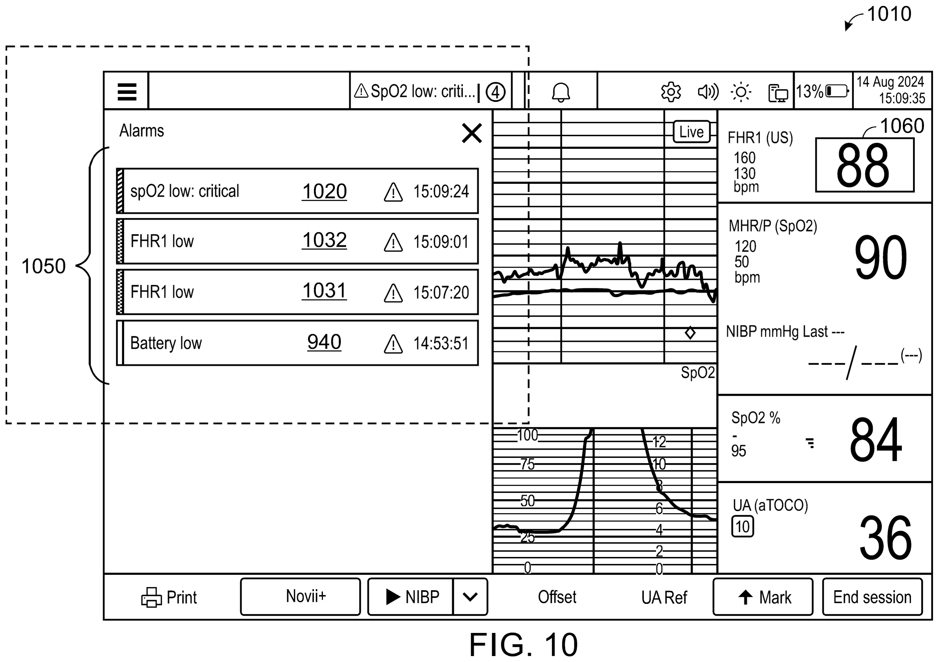

Generation of alarms may include a visual alert on a display, a push notification alert to one or more caregivers, an auditory alert, or all of the above. In some embodiments, an auditory alert will be generated for only one of the alarms (e.g., the current or more recent alarm) and one or more visual alerts are generated for all the alarms. FIG. 10 illustrates another exemplary user interface arrangement showing the ordered list of alarms for a given fetal patient where alarms for both latched and unlatched fetal parameters are included. The list of fetal alarms 1050 for the fetal patient includes two alarms for the first fetal parameter (here, two fHR low alarms 1031 and 1032 for the fHR fetal parameter) are included. Fetal SpO₂ is also a monitored fetal parameter, which may be a latched parameter monitored according to the disclosed methods, or may be an unlatched parameter.

The list of fetal alarms 1050 for the fetal patient includes an SpO₂ low alarm 1020. Additionally, the list of fetal alarms 1050 may include technical alarms or other informational alarms about the sensors or patient monitoring system for that patient. In the depicted example, the list of fetal alarms 1050 for the fetal patient includes a technical alarm 1040 indicating that a sensor battery is low, e.g., on a wireless sensor or monitoring hub on the fetal patient. Other technical alarms may include sensor off alarms, alarms relating to signal strength, or other alarms relating to technical functionality of one or more patient-specific devices.

The list of fetal alarms 1050 for the first fetal patient is displayed on the user interface display 1010. The list of fetal alarms 1050 is displayed in such a way as to provide visual alert of each alarm. The user interface display 1010 may include one or more additional visual alerts for one or more of the alarms. For example, an additional alarm indicator 1060 may be displayed for the most recent (or current) alarm for each alarming physiological parameter. Alternatively or additionally, an auditory alert may be generated for one or more of the alarming parameters. In some embodiments, an auditory alert will be generated for only one of the alarms (e.g., the current or most recent alarm).

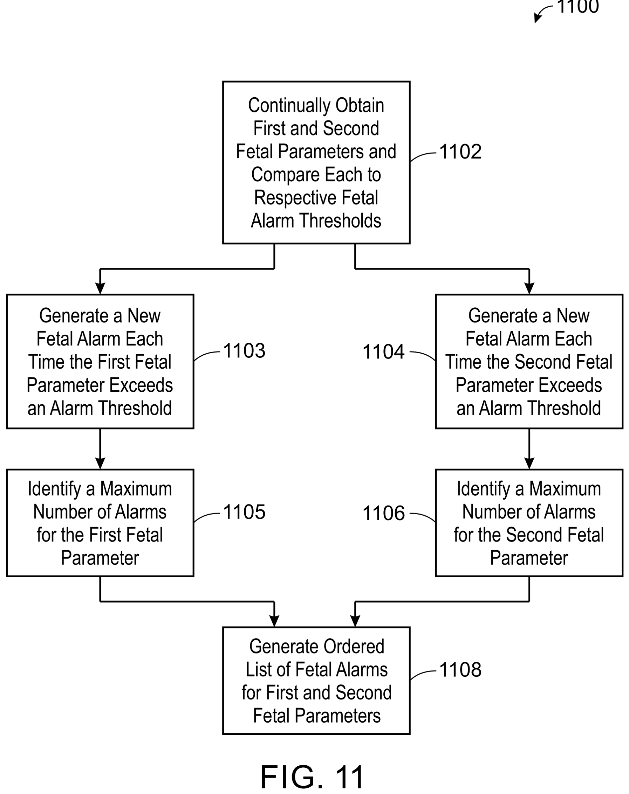

FIG. 11 shows another embodiment of a method of processing the fetal physiological parameters for purposes of maternal and fetal monitoring. The method 1100 is configured for processing two fetal parameters for a first fetal patient where both are latched parameters where the list of alarms is generated according to the present disclosure. The first and second fetal parameters are continually obtained by sensors on the first patient and each compared to one or more respective fetal alarm thresholds, represented as step 1102. A new fetal alarm for the first parameter is generated at step 1103 each time the first fetal parameter exceeds an alarm threshold. A new fetal alarm for the second parameter is generated at step 1104 each time the second fetal parameter exceeds an alarm threshold. Where both the first and second fetal parameters are latched parameters with alarm list generated according to the disclosed methods, logic is executed at steps 1105 and 1106 to identify a maximum number of alarms for each of the first and second fetal parameters. The maximum number of alarms for the first fetal parameter may be the same or different than the maximum number of alarms for the second fetal parameter. The fetal alarms for the first and second fetal parameters are then ordered, such as based on alarm level and/or alarm time. The ordered list of fetal alarms for the first fetal patient is then generated at step 1108.

Where multiple patients are monitored, including the first fetal patient, a maternal patient, and/or one or more additional fetal patient(s), a separate list of alarms may be generated for each patient. The lists of alarms may include all alarms for the respective patient, which may include a plurality of latched parameters with multiple alarms per parameter, as disclosed herein, and also may include unlatched parameter alarms and/or technical alarms for that patient.

In various embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A method of monitoring depth of muscle relaxation of a patient, the method comprising:
applying a series of stimulations to a nerve of a patient and measuring muscle responses thereto;
based on the muscle responses, determining at least one of a maximal stimulus current, a supramaximal stimulus current, and a submaximal stimulus current for the patient, wherein the maximal stimulus current is a current at which a maximal muscle response is produced from stimulating the nerve, the supramaximal stimulus current is greater than or equal to the maximal stimulus current, and the submaximal stimulus current is less than the maximal stimulus current;
applying a first set of stimulations to the nerve of the patient and measuring muscle responses to the first set of stimulations; and
selecting one of the supramaximal stimulus current and the submaximal stimulus current for a subsequent series of stimulations based on the measured muscle responses to the first set of stimulations; and
applying the subsequent series of stimulations at the selected stimulus current to the nerve of the patient and measuring muscle responses thereto.

2. The method of claim 1, wherein the first set of stimulations are a series of supramaximal stimulations and further comprising determining whether the measured muscle responses to the first set of stimulations are at least equal to a supramax response threshold, wherein selecting one of the supramaximal stimulus current and a submaximal stimulus current for a subsequent series of stimulations includes:
if the measured muscle responses are at least equal to the supramax response threshold, then selecting the submaximal stimulus current for the subsequent series of stimulations; and
if the measured muscle responses are less than the supramax response threshold, then selecting the supramaximal stimulus current for the subsequent series of stimulations.

3. The method of claim 2, when the submaximal stimulus current for the subsequent series of stimulations is selected, further comprising periodically applying the series of submaximal stimulations to the nerve of the patient at the submaximal stimulus current and measuring submax muscle responses to monitor the patient's depth of muscle relaxation.

4. The method of claim 3, further comprising identifying that the measured submax muscle responses are less than a submax response threshold, and then selecting the supramaximal stimulus current for a next series of stimulations.

5. The method of claim 4, further comprising generating a recurarization alarm upon identifying that the measured submax muscle responses are less than the submax response threshold.

6. The method of claim 4, further comprising:
applying the next series of stimulations at the supramaximal stimulus current and measuring muscle responses thereto;
comparing the measured muscle responses to the supramax response threshold to confirm or reject recurarization;
if recurarization is confirmed, generating a recurarization alarm; and
if recurarization is rejected, then selecting the submaximal stimulus current for the next series of stimulations.

7. The method of claim 2, where the series of stimulations are train-of-four (TOF) stimulations, and wherein the measured muscle responses are four responsive muscle twitches.

8. The method of claim 7, further comprising:
determining a TOF response based on the measured muscle responses;
wherein determining whether the measured muscle responses are at least equal to the supramax response threshold includes comparing the TOF response to the supramax response threshold, and then selecting one of the supramaximal stimulus current and a submaximal stimulus current for a subsequent series of stimulations based on a result of the comparison.

9. The method of claim 1, wherein the supramaximal stimulus current is a preselected percentage greater than the maximal stimulus current determined for the patient, and wherein the submaximal stimulus current is a preselected percentage less than the maximal stimulus current or the supramaximal stimulus current.

10. A system for monitoring depth of muscle relation of a patient, the system comprising:
a stimulator configured to apply a series of stimulations to a nerve of the patient;
a sensor device configured to measure muscle responses of the patient to the series of stimulations;
a storage medium storing a stimulation mode indicator that indicates a stimulation mode for the stimulator and/or a stimulus current indicator of at least one of a maximal stimulus current, a supramaximal stimulus current, and a submaximal stimulus current for the patient, wherein the maximal stimulus current is a current at which a maximal muscle response is produced from stimulating the nerve, the supramaximal stimulus current is greater than or equal to the maximal stimulus current, and the submaximal stimulus current is less than the maximal stimulus current;
a controller configured to:
control the stimulator to apply a set of stimulations to the nerve of the patient at varying stimulus currents and measure muscle responses to the set of stimulations to determine at least one of the maximal stimulus current, the supramaximal stimulus current, and a submaximal stimulus current for the patient;
control the stimulator to periodically apply a TOF stimulation to the nerve of the patient at the supramaximal stimulus current and measure a TOF response to the supramaximal stimulations;
compare the measured TOF response to a supramax response threshold;
upon identifying that the measured TOF response is at least the supramax response threshold:
periodically apply a TOF stimulation to the nerve of the patient at the submaximal stimulus current and measure a submax TOF responses to monitor the depth of muscle relaxation of the patient; and
store a submaximal stimulation mode indicator in the storage medium.

11. The system of claim 10, wherein the controller is integrated into a connector connecting the sensor device to a patient monitor, and wherein the controller is configured to receive the stimulation mode indicator and/or a stimulus current indicator stored in the storage medium upon connection of the sensor device to the patient monitor.

12. The system of claim 10, wherein the controller is integrated into a connector connecting the sensor device to a patient monitor, and further comprising:
a wireless transceiver in the connector;
wherein the controller is further configured to wirelessly connect to a patient monitor configured to monitor the patient.

13. The system of claim 10, wherein the storage medium is integrated into the sensor device, and wherein the controller is configured to receive the stimulation mode indicator and/or a stimulus current indicator stored in the storage medium upon connection of the sensor device to the patient monitor.

14. The system of claim 10, wherein the controller is further configured to:
identify that the submax TOF response is less than a submax response threshold, and then reapply the TOF at the supramaximal stimulus current and measuring a new TOF response thereto; and
compare the new TOF response to the supramax response threshold to confirm or reject recurarization.

15. The system of claim 14, wherein the controller is further configured to generate a recurarization alarm upon identifying that the submax TOF response is less than the submax response threshold and/or upon confirming recurarization.
